# EUROPÄISCHE PATENTSCHRIFT

(11) **EP 1 725 301 B1**
(45) Veröffentlichungstag und Bekanntmachung des Hinweises auf die Patenterteilung: **09.05.2012**
(21) Anmeldenummer: 05729834.1
(22) Anmeldetag: 11.03.2005
(51) Int. Cl.: A61K 8/29, A61Q 17/04

(54) **KOSMETISCHES PR PARAT MIT UV-SCHUTZ UND VERWENDUNG VON EFFEK TPIGMENTEN MIT SCHUTZSCHICHT**
UV-PROTECTIVE COSMETIC PREPARATION AND USE OF DECORATIVE PIGMENTS COMPRISING A PROTECTIVE LAYER
PREPARATION COSMETIQUE A ECRAN U.V. ET UTILISATION DE PIGMENTS A EFFET PRESENTANT UNE COUCHE PROTECTRICE

(30) Priorität: 19.03.2004 DE 102004014020
(43) Veröffentlichungstag der Anmeldung: 29.11.2006
(73) Patentinhaber: Sudarshan Chemical Industries Ltd., Pune 411 001 MAH (IN)
(72) Erfinder: SCHUSTER, Thomas, 91207 Lauf (DE); KRÜGER, Peter, 91207 Lauf (DE); SCHMIDT, Ulrich, 91217 Hersbruck (DE)
(74) Vertreter: Walcher, Armin
(86) Internationale Anmeldenummer: PCT/EP2005/002615
(87) Internationale Veröffentlichungsnummer: WO 2005/092281

(56) Entgegenhaltungen:
- EP-A- 0 224 978
- EP-A- 0 665 004
- EP-A- 0 753 545
- EP-A- 0 898 955
- EP-A- 1 213 006
- WO-A-2004/026268
- WO-A-2004/100922
- DE-A1- 2 313 331
- DE-A1- 2 603 211
- DE-A1- 3 137 808
- DE-A1- 3 137 809
- DE-A1- 3 151 343
- DE-A1- 3 151 355
- DE-A1- 3 334 598
- DE-A1- 10 034 332
- DE-A1- 19 907 313
- PATENT ABSTRACTS OF JAPAN Bd. 1998, Nr. 01, 30. Januar 1998 (1998-01-30) & JP 09 227114 A (MORI SADAYOSHI), 2. September 1997 (1997-09-02)
- YAMAMOTO MASARU ET AL: "UV-SHIELDING COMPOSITE MICA POWDERS AND PEARLY PIGMENTS" CHEMICAL ABSTRACTS, 9. Oktober 1995 (1995-10-09), XP002177803

## Beschreibung

Die Erfindung betrifft ein kosmetisches Präparat mit UV-Schutz sowie die Verwendung von Effektpigmenten mit wenigstens einer Schutzschicht zur Herstellung eines kosmetischen Präparats mit UV-Schutz.

Kosmetische Präparate wie Sonnenschutzmittel finden im alltäglichen Leben breite Verwendung. Insbesondere im Hinblick auf die in den letzten Jahren bekannt gewordenen schädigenden Einflüsse von UV-Licht auf die menschliche Haut wird eine Vielzahl von Sonnenschutzmitteln auf dem Markt angeboten. Die Entwicklung geht hin zu immer höheren Lichtschutzfaktoren, um dem Bedürfnis nach einem verlässlichen Schutz trotz langer Verweilzeiten im Sonnenlicht gerecht zu werden.

Das schädigende UV-Licht wird in die Bereiche UV-A (320 bis 400 nm) und UV-B (280 bis 320 nm) eingeteilt. Das UV-B-Licht ist aufgrund seiner kürzeren Wellenlänge energiereicher. Eine übermäßige Exposition von ungeschützter menschlicher Haut gegenüber UV-Licht kann zu Hautkrebs führen.

Als UV-A-Absorber wirken beispielsweise Benzophenone oder Avobenzon. Typische UV-B-Absorber sind p-Aminobenzoesäure oder Cinnamate. Kampferderivate können abhängig von ihrer Substitution eine Breitbandwirkung im gesamten UV-Bereich aufweisen.

Neben UV-Absorbern können Sonnenschutzmittel auch Pigmente enthalten. Diese können als physikalischer UV-Schutz wirken, indem sie die Haut abdecken, d.h. das UV-Licht abschirmen.

Die DE 25 44 180 offenbart ein Sonnenschutzmittel, das 4-Isopropyldibenzoylmethan als UV-Absorber enthält.

Die CH 11639/78 bzw. die US 4,387,089 offenbaren die Verwendung von 4-(1,1-Dimethylethyl)-4'-methoxydibenzoylmethan als UV-A-Absorber, das sich auch auf die Wirkung von UV-B-Absorbern günstig auswirken soll.

In der DE 33 02 123 wird 2,4-Dimethyl-4'-methoxy-dibenzoylmethan und ein Sonnenschutz, der diesen Stoff enthält, beschrieben.

Gegenstand der US 6,210,658 ist ein Sonnenschutzmittel, das UV-A-, UV-B-Absorber sowie eine Bariumverbindung zur Stabilisierung enthält.

Die DE 41 23 772 und die EP 1 078 883 B1 offenbaren die Verwendung von pyrogen hergestelltem Titandioxid in Sonnenschutzmitteln.

Die EP 1 078 957 B1 offenbart die Verwendung von oberflächenmodifiziertem, pyrogen hergestelltem Titandioxid in Sonnenschutzmitteln.

Bei der Verwendung von Titandioxidpartikeln in Sonnenschutzmitteln ist nachteilig, dass nach Aufbringung des Sonnenschutzmittels auf der Haut häufig ein weißer Schleier erzeugt wird, der wenig attraktiv wirkt.

Es könnte daran gedacht werden, einem Sonnenschutzmittel Perlglanzpigmente zuzusetzen, um nach dem Auftragen des Sonnenschutzmittels auf der Haut einen dekorativen Effekt zu erzeugen.

Es hat sich jedoch gezeigt, dass unter Einwirkung von Sonnenlicht bei Perlglanzpigmenten ein Abbau der UV-Absorber des kosmetischen Präparats auftreten kann, was äußerst unerwünscht ist, da hierdurch der UV-Schutz zerstört wird. Bedingt durch die Zerstörung des UV-Schutzes, ist es sodann erforderlich, das kosmetische Präparat, beispielsweise ein Sonnenschutzmittel, in relativ kurzen Zeitabständen erneut auf die Haut aufzutragen, um einer Schädigung der Haut durch UV-Licht vorzubeugen.

Es besteht insbesondere beim modebewussten Verbraucher der Wunsch nach Kosmetika; die auf der einen Seite die Haut mit einem lang anhaltenden UV-Schutz versehen und auf der anderen Seite einen dekorativen Zweck erfüllen.

Die der Erfindung zugrundeliegende Aufgabe wird durch Bereitstellung eines kosmetischen Präparats mit UV-Schutz gemäss Anspruch 1, enthaltend einen oder mehrere organische UV-Absorber, gelöst, wobei das kosmetische Präparat Perlglanzpigmente enthält und wobei die Perlglanzpigmente wenigstens eine Schutzschicht gemäss Anspruch 1 aufweisen.

Bevorzugte Weiterbildungen sind in den Unteransprüchen 2 bis 6 angegeben.

Die der Erfindung zugrundeliegende Aufgabe wird weiterhin durch die Verwendung von Perlglanzpigmenten gemäss Anspruch 7 mit wenigstens einer Schutzschicht zur Herstellung eines kosmetischen Präparats mit UV-Schutz gelöst.

Bevorzugte Weiterbildungen sind in den Unteransprüchene 2 bis 10 angegeben.

Es hat sich überraschend gezeigt, dass Perlglanzpigmente durch das Aufbringen wenigstens einer Schutzschicht aus Siliciumdioxid so stabilisiert werden können, dass kein bzw. nur ein unwesentlicher Abbau von UV-Absorbern in einem kosmetischen Präparat bei Einstrahlung von Sonnenlicht erfolgt.

Das erfindungsgemäße kosmetische Präparat mit UV-Schutz, das zusätzlich Perlglanzpigmente gemäss Anspruch 1 enthält ermöglicht neben der Bereitstellung eines langhaltenden UV-Schutzes die Erzeugung vielfältiger Farbeffekte auf der Haut. Die Perlglanzpigmente gemäss Anspruch 1 richten sich aufgrund ihrer flächigen Struktur im wesentlichen parallel zur Hautoberfläche aus. Insbesondere bei Verwendung von Perlglanzpigmenten mit Schutzschicht lassen sich in Abhängigkeit von dem Schichtenaufbau interessante Farbeffekte auf der Haut hervorrufen. Bei Perlglanzpigmenten werden zur Erzeugung der Farbeffekte durch Interferenz in der Regel auf Glimmerpartikeln eine Schicht oder mehrere Schichten aus Metall und/oder Metalloxiden aufgebracht.

Je nach Einfallswinkel des Lichtes und Schichtenaufbau der Pigmente erscheint die Haut dann mit einer anderen Farbe oder einem anderen Farbton. Diese Glanz- und Farbeffekte verleihen der Haut ein interessantes Aussehen. Des weiteren können mit dem erfindungsgemäßen Kosmetikpräparat auch unschöne Bereiche, wie beispielsweise Pigmentierungsstörungen in der Haut, von Akne befallene Hautpartien, Narben, etc. abgedeckt und mithin geschönt werden.

Vorzugsweise ist die wenigstens eine Schutzschicht im wesentlichen transparent. Die - vorzugsweise im wesentlichen transparente - Schutzschicht umhüllt die Effektpigmente vollständig.

Erfindungsgemäss wird eine Schutzschicht aus SiO₂ aufgebracht. Die Umhüllung von Perlglanzpigmenten mit einer SiO₂-Schicht kann auf einfache Art und Weise, beispielsweise durch Behandlung mit Wasserglas, erfolgen. Eine Schutzschicht aus SiO₂ ist im wesentlichen transparent. Insofern beeinflusst eine Schutzschicht aus SiO₂ den durch auf dem Pigment aufgebrachte weitere Metall- und/oder Metalloxidschichten erzeugten Farbeffekt nicht bzw. unwesentlich.

Eine solche Schutzschicht sollte eine Isolation des Perlglanzpigmentes gegenüber der Umgebung bewirken. Unter Umgebung wird vorliegend das kosmetische Präparat mit den organischen UV-Absorbern verstanden, in das die Perlglanzpigmente eingemengt sind.

Die vorliegende Erfindung ermöglicht es mithin, kosmetische Präparate oder Mittel mit einem UV-Absorber oder mehreren UV-Absorbern bereitzustellen, die zusätzlich Perlglanzpigmente gemäss Anspruch 1 enthalten und die auch bei Belichtung bzw. Einstrahlung von Sonnenlicht langandauernd stabil sind. Das heißt, bei Verwendung von Perlglanzpigmenten gemäss Anspruch 1 in einem kosmetischen Präparat mit UV-Schutz erfolgt kein merklicher Abbau des organischen UV-Absorbers bzw. der organischen UV-Absorber.

Es hat sich überraschend gezeigt, dass Perlglanzpigmente, die eine TiO₂-Beschichtung aufweisen, durch wenigstens eine Schutzschicht, vorzugsweise anorganische Schutzschicht, so stabilisiert werden können, dass das TiO₂ keinen Abbau von organischen UV-Absorbern induziert.

Durch die anorganische Schutzschicht aus Siliciumdioxid wird das TiO₂ bzw. die TiO₂-Schicht gegenüber der Umgebung isoliert, so dass kein Abbau von organischen UV-Absorbern erfolgt.

Das erfindungsgemäße kosmetische Präparat, vorzugsweise ein Sonnenschutzmittel, enthält in einem geeigneten und üblichen Trägersystem, das dem Fachmann bekannt ist, einen oder mehrere organische UV-Absorber.

Der UV-Absorber wird vorzugsweise aus der Gruppe, die aus Benzophenonen, Hydroxynaphthochinonen, Phenylbenzoxazolen, Phenylbenzimidazolen, Digalloyltrioleat, Aminobenzoesäureestem, Salicylsäureestem alicyclischen Dienonen, Zimtsäureestern, Benzalazin, Avobenzon, Paraaminobenzoesäure und -derivaten, Cinnamaten, Salicylaten, Kampferderivaten, Benzimidazolen, 4-Isopropyldibenzoylmethan, 4-(1,1-Dimethylethyl)-4'-methoxydibenzoylmethan, 2,4-Dimethyl-4'-methoxydibenzoylmethan und Gemischen davon besteht, ausgewählt.

Erfindungsgmäss enthält das Perlganzpigment Titandioxid. Vorzugsweise weist das Perlglanzpigment einen Schichtenaufbau auf, bei dem, direkt oder indirekt, über der TiO₂-Schicht wenigstens eine Schutzschicht aus Siliciumdioxid aufgebracht ist.

Die Schutzschicht des Perlglanzpigments wird vorzugsweise in einem wässrigen System, beispielsweise unter Verwendung von Wasserglas, aufgebracht.

Das kosmetische Präparat oder Mittel kann in Form einer Creme, Lotion, Milch, Emulsion, Sprayemulsion, eines Gelees, Öls, Sprayöls oder Aerosols vorliegen. Vorzugsweise ist das kosmetische Präparat ein Sonnenschutzmittel.

Die nachstehenden Beispiele dienen der weiteren Veranschaulichung der Erfindung. Die Erfindung ist jedoch nicht auf diese Beispiele beschränkt.

### Beispiel 1: Beschichtung eines Perlglanzpigments

Zu einer 10 %igen Suspension Perlglanzpigment (Prestige Silver der Fa. ECKART, Fürth, Deutschland) wird bei 75 °C eine Lösung von Natronwasserglas 37/40 BE (11 g Natronwasserglas in 15 g Wasser) innerhalb von 15 min komplett eingeleitet, der pH wird dabei nicht kontrolliert. Nach beendeter Zugabe wird der pH-Wert mit verdünnter Salzsäure auf pH 7.5 gesenkt. Die Suspension wird anschließend 1 h bei konstantem pH-Wert nachgerührt, über einen Büchnertrichter abfiltriert, mit 1000 ml Wasser gewaschen und über Nacht bei 120°C im Trockenschrank getrocknet.

### Beispiel 2: Beschichtung eines Perlglanzpigments

Zu einer 10 %igen Suspension Perlglanzpigment (Prestige Silver Star der Fa. ECKART, Fürth, Deutschland) wird bei 75°C eine Lösung von Natronwasserglas 37/40 BE (11 g Natronwasserglas in 15 g Wasser) innerhalb von 15 min komplett eingeleitet, der pH wird dabei nicht kontrolliert. Nach beendeter Zugabe wird der pH-Wert mit verdünnter Salzsäure auf pH 7.5 gesenkt. Die Suspension wird anschließend 1 h bei konstantem pH-Wert nachgerührt, über einen Büchnertrichter abfiltriert, mit 1000 ml Wasser gewaschen und über Nacht bei 120°C im Trockenschrank getrocknet.

### Beispiel 3: Herstellung einer Feuchtigkeitscreme mit Perlglanzpigmenten

Phase 1 und Phase 2, die jeweils eine Zusammensetzung aufwiesen, wie in Tabelle 1 bzw. Tabelle 2 angegeben, wurden jeweils auf 78° C erwärmt. Danach wurde Phase 2 zu Phase 1 unter Homogenisieren zugegeben. Nachfolgend ließ man die Mischung unter Rühren auf Raumtemperatur unter Erhalt einer Creme abkühlen.

In einem ersten Ansatz wurden 19.4 g dieser Creme sodann mit 0.6 g Prestige Silver Star-Pigmenten, die eine Beschichtung mit 2.5 Gew.-% SiO₂ aufwiesen, vermengt.

In Entsprechung zu dem ersten Ansatz wurden in einem zweiten Ansatz ebenfalls 19,4 g der Creme mit 0,6 g Prestige Silver Star-Pigmenten, die jedoch nicht mit SiO₂ beschichtet waren, unter gleichen Bedingungen vermengt.

Schließlich wurden beide Ansätze gegenüber Sonnenlicht unter identischen Bedingungen exponiert. Im Vergleich zu einer Creme mit Prestige Silver Star ohne Si02-Beschichtung zeigte sich, dass der Abbau des Avobenzons stark verringert war.

**Tabelle 1: Zusammensetzung von Phase I**

| INCI-Name | Produktbezeichnung | Gew.-% | Lieferant |
|---|---|---|---|
| **Phase I:** | | | |
| | | | |
| Cyclomethicon | Dow Corning 345 Fluid | 17.86% | Dow Corning |
| Dimethicon | Dow Corning 200 Fluid, 350 CST | 4.96% | Dow Corning |
| C12-C15 Alkylbenzoat (und) Stearalkoniumbentonit (und) Propylencarbonat | Tixogel FTN | 9.92% | Süd-Chemie Rheologicals |
| Cetyldimethicon-Copolyol | Abil EM 90 | 1.19% | Degussa |
| Butylmethoxydibenzoylmethan (Avobenzon) | Parsol 1789 | 2.48% | Roche |

**Tabelle 2: Zusammensetzung von Phase II**

| **Phase II:** | | | |
|---|---|---|---|
| | | | |
| INCI-Name | Produktbezeichnung | Gew.-% | Lieferant |
| | | | |
| Entionisiertes Wasser | | 62.62% | |
| Natriumchlorid | | 0.37% | |
| Phenoxyethanol (und) Methylparaben (und) Butylparaben (und) Propylparaben | Uniphen P-23 | 0.60% | Lipo Chemical |

## Patentansprüche

1. Kosmetisches Präparat mit UV-Schutz, enthaltend einen oder mehrere organische UV-Absorber,
**dadurch gekennzeichnet,**
**dass** das kosmetische Präparat Perlglanzpigmente enthält, wobei die Perlglanzpigmente wenigstens eine die Perlglanzpigmente umhüllende Schutzschicht aus Siliciumdioxid aufweisen und die Perlglanzpigmente Titandioxid enthalten, wobei die umhüllende Schutzschicht das TiO₂ bzw. die TiO₂ - Schicht gegenüber der Umgebung isoliert, so dass kein Abbau von organischen Verbindungen des kosmetischen Präparats - erfolgt.

2. Kosmetisches Präparat nach Anspruch 1,
**dadurch gekennzeichnet,**
**dass** die Schutzschicht im wesentlichen transparent ist.

3. Kosmetisches Präparat nach Anspruch 1 oder 2,
**dadurch gekennzeichnet,**
**dass** der organische UV-Absorber aus der Gruppe, die aus Benzophenonen, Hydroxynaphthochinonen, Phenylbenzoxazolen, Phenylbenzimidazolen, Digalloyltrioleat, Aminobenzoesäureestern, Salicylsäureestern alicyclischen Dienonen, Zimtsäureestern, Benzalazin, Avobenzon, Paraaminobenzoesäure und -derivaten, Cinnamaten, Salicylaten, Kampferderivaten, Benzimidazolen, 4-
Isopropyldibenzoylmethan, 4-(1,1-Dimethylethyl)-4'-methoxydibenzoylmethan, 2,4-Dimethyl-4'-methoxy-dibenzoylmethan und Mischungen davon besteht, ausgewählt wird.

4. Kosmetisches Präparat nach einem der vorherigen Ansprüche,
**dadurch gekennzeichnet,**
**dass** die Perlglanzpigmente einen Schicht-Substrat-Aufbau aufweisen.

5. Kosmetisches Präparat nach einem der Ansprüche 1 bis 4,
**dadurch gekennzeichnet,**
**dass** es in Form einer Creme, Lotion, Milch, Emulsion, Sprayemulsion, eines Gelees, Öls, Sprayöls oder Aerosols vorliegt.

6. Kosmetisches Präparat nach einem der vorherigen Ansprüche,
**dadurch gekennzeichnet,**
**dass** es ein Sonnenschutzmitel ist.

7. Verwendung von Perlglanzpigmenten zur Herstellung eines kosmetischen Präparats mit UV-Schutz enthaltend einen oder meheren organisch UV-Absorber, wobei die Perlglanzpigmente wenigstens eine die Perlglanzpigmente umhüllende Schutzschicht aus Siliciumdioxid aufweisen und die Perlglanzpigmente Titandioxid enthalten, wobei die Schutzschicht das TiO₂ bzw. die TiO₂ - Schicht gegenüber der umgebung isoliert, so dass kein Abbau von organischen Verbindungen des kosmatischen Präparats erfolgt.

8. Verwendung nach Anspruch 7,
**dadurch gekennzeichnet,**
**dass** die Schutzschicht im wesentlichen transparent ist,

9. Verwendung nach Anspruch 7 oder 8,
**dadurch gekennzeichnet,**
**dass** die Perlglanzpigmente einen Schicht-Substrat-Aufbau besitzen.

10. Verwendung nach einem der Ansprüche 7 bis 9,
**dadurch gekennzeichnet,**
**dass** das kosmetische Präparat ein Sonnenschutzmittel ist.

## Claims

1. Cosmetic preparation with UV- protection containing one or more organic UV- absorber,
**characterized by that**,
that the cosmetic preparation contains pearl effect pigments, wherein the pearl effect pigments comprise at least one protective coating surrounding the pearl effect pigments, made of Silicium dioxide and which contain the Titanium dioxide, wherein the surrounding protective coating isolates the TiO₂ or the TiO₂- coating from the surroundings, such that no formation of the organic compounds of the cosmetic preparation takes place.

2. Cosmetic preparation according to claim 1,
**characterized by that**,
that the protective coating is essentially transparent.

3. Cosmetic preparation according to claim 1 or 2,
**characterized by that**,
that the organic UV- absorber is selected from the group, which consists of benzophenones, hydroxyl naphthoquinones, phenyl benzoxazoles, phenyl benzimidazoles, digalloyl trioleate, aminobenzoic acid esters, salicylic acid esters, alicyclic dienones, cinnamic acid esters, benzalazin, avobenzon, paraaminobenzoic acid esters and derivatives, cinnamates, salicylates, camphor derivatives, benzimidazoles, 4- isopropyl dibenzoylmethane, 4- (1, 1- dimethylethyl) 4'- methoxy dibenzoyl methane, 2,4- dimethyl-4'-methoxy-dibenzoyl methane and mixtures thereof

4. Cosmetic preparation according to one of the preceding claims,
**characterized by that**,
that the pearl effect pigments comprise a coating- substrate structure.

5. Cosmetic preparation according to one of the claims 1 to 4,
**characterized by that**,
that it is present in the form of a cream, lotion, emulsion, spray emulsion, a gel, oil, spray oil or aerosol.

6. Cosmetic preparation according to one of the preceding claims,
**characterized by that**,
that it is a sun protection medium.

7. Use of pearl effect pigments for manufacturing a cosmetic preparation with UV-protection containing one or more organic UV- absorber, wherein the pearl effect pigments comprise at least one protective coating surrounding the pearl effect pigments, made of Silicium dioxide and which contain the Titanium dioxide, wherein the protective coating isolates the TiO₂ or the TiO₂- coating from the surroundings, such that no formation of the organic compounds of the cosmetic preparation takes place.

8. Use according to claim 7,
**characterized by that**,
that the protective coating is essentially transparent.

9. Use according to claims 7 or 8,
**characterized by that**,
that the pearl effect pigments have a coating- substrate structure.

10. Use according to one of the claims 7 to 9,
**characterized by that**,
that the cosmetic preparation is a sun- protection medium.

## Revendications

1. Le Produit cosmétique avec une protection UV comprennant un ou plusieurs d'absorbeur d'UV organique, **caractérisé en ce que**, le produit cosmétique compris les pigments à effets nacrés, dans lesquels les pigments à effets nacrés comprennent au moins un revêtement protecteur entourant les pigments à effets nacrés composé de dioxyde de silicium et comprennant le dioxyde de titane, dans lequel le revêtement protecteur entourant isole le revêtement du TiO2 ou TiO2 de l'environnement de sorte qu'il n'y aucune formation de composés cosmétiques du produit cosmétique.

2. Le Produit cosmétique selon le revendication 1, **caractérisé en ce que**, le revêtement protecteur est essentiellement transparent.

3. Le Produit cosmétique selon le revendication 1, **caractérisé en ce que**, l'absorbeur d'UV organique est choisi parmi le groupe comprennant le benzophénones, l'hydroxylnaphthoquinones, des phénylbenzoxazoles, des phényl benzimidazoles, le trioléate de digalloyl, l'ester d'acide aminobenzoïque , des esters d'acide salicylique, d'acides alicyliques, des esters d'acide cinnamique *benzalazin, avobenzon,* des esters d'acide paraaminobenzoïque et ces dérivés, des cinnamates, des salicylates, des dérivés du camphre, des benzimidazoles, 4- dibenzoylméthane isopropylique, 4- (1, 1- diméthyléthyle) 4'- méthoxy dibenzoylméthane, 2,4- diméthyle -4'-méthoxy - dibenzoylméthane et mélanges de ces substances.

4. Le Produit cosmétique selon l'une des revendications précédentes, **caractérisé en ce que**, les pigments à effets nacrés comprennent une structure du revêtement d'un substrat.

5. Le Produit cosmétique selon l'une des revendications 1 à 4, **caractérisé en ce qu'**il est sous la forme d'une crème, d'une lotion, d'une émulsion , d'une émulsion à vaporisateur, un gel, des huille, des huile de pulvérisation ou d'aérosols.

6. Le Produit cosmétique selon l'une des revendications précédentes, **caractérisé en ce qu'**il est un moyen de protection contre le soleil.

7. L'utilisation de pigments à effets nacrés pour la fabrication d'un produit cosmétique avec une protection UV comprennant un ou plusieurs d'absorbeur d'UV organique, dans lesquels les pigments à effets nacrés comprennent au moins un revêtement protecteur entourant les pigments à effets nacrés composé de dioxyde de silicium et comprennant le dioxyde de titane, dans lequel le revêtement protecteur entourant isole le revêtement du Ti02 ou Ti02 de l'environnement de sorte qu'il n'y aucune formation de composés cosmétiques du produit cosmétique..

8. L'utilisation selon le revendication 7, **caractérisé en ce que**, le revêtement protecteur est essentiellement transparent.

9. L'utilisation selon les revendications 7 ou 8, **caractérisé en ce que**, les pigments à effets nacrés ont une structure du revêtement d'un substrat.

10. L'utilisation selon les revendications 7 à 9, **caractérisé en ce que**, le produit cosmétique est un moyen de protection contre le soleil.
